Europäisches Patentamt

European Patent Office

Office européen des brevets

(11) Publication number: **0 316 170**
**A2**

# EUROPEAN PATENT APPLICATION

(21) Application number: **88310591.8**

(22) Date of filing: **10.11.88**

(51) Int. Cl.4: **C 12 Q 1/68**
C 12 N 15/00, C 12 Q 1/70,
C 07 H 21/04, C 07 K 13/00,
G 01 N 33/569, A 61 K 39/245

(30) Priority: **11.11.87 IE 3041/87**

(43) Date of publication of application:
**17.05.89 Bulletin 89/20**

(84) Designated Contracting States:
**AT BE CH DE ES FR GB GR IT LI LU NL SE**

(71) Applicant: **G GENE GALWAY LIMITED**
**University College Galway**
**Galway (IE)**

(72) Inventor: **Gannon, Bernard Francis Xavier**
**22, Pollnarooma West**
**Salt Hill Galway (IE)**

**Walls, Dermot Felix Gerard**
**Dept. of Microbiology University College Galway**
**Galway (IE)**

(74) Representative: **Goldin, Douglas Michael et al**
**J.A. KEMP & CO. 14, South Square Gray's Inn**
**London WC1R 5EU (GB)**

(54) Method for the identification of antigens expressed in vivo, diagnostic assays therefor and vaccines containing them.

(57) A method for identifying all of the antigens from a pathogenic organism such as Epstein-Barr Virus which are expressed in vivo comprises generating a gene bank using nucleic acid fragments which include all of the nucleic acid from a given organism and direct screening of said gene bank with serum from a subject with clinical symptoms caused by the pathogenic organism. The antigens identified can be used as a basis for various diagnostic assays and for preparing vaccines.

EP 0 316 170 A2

**Description**

# METHOD FOR THE IDENTIFICATION OF ANTIGENS EXPRESSED IN VIVO, DIAGNOSTIC ASSAYS THEREFOR AND VACCINES CONTAINING THEM

This invention relates to antigens which are expressed in vivo and to their use in diagnostic assays and as vaccines. More particularly, the invention relates to viral antigens, especially Epstein-Barr Virus (EBV) antigens, and to their use in diagnostic assays and as vaccines.

The importance of the provision of purified antigens for use in diagnostic tests is widely accepted. Traditionally, purified antigens have been obtained by extensive purification using biochemical methods. The alternative of using recombinant DNA technology to clone and express an isolated antigen in a host organism is now used in an increasing number of situations.

The Epstein-Barr Virus is a herpes virus ubiquitous for humans. EBV is the causative agent of infectious mononucleosis (IM). It may be involved in rheumatoid arthritis and is an etiologic agent of some B-lymphomas such as Burkitt's lymphoma and nasopharyngeal carcinoma. EBV may also have more benign effects with the result that much of the population may have sera which is positive for antibodies to EBV without having a medical history which would include any of the aforementioned ailments.

In vitro, EBV stimulates the growth of B-lymphocytes and may exist in a productive or latent state.

The diagnosis of EBV infection or reactivation is usually carried out by a heterophyl Paul-Bunnel-Davidson (Monospot) test in which a fortuitous cross reactivity between the EBV antibodies and an unrelated horse protein is used. Although this method is cheap and quick, it is clearly useful only as a crude primary screen because of its basis and the fact that it is only approximately 80% effective. The method is not 100% effective largely because of the fact that EBV infection has different phases and is abnormal in progression. Hence there is a requirement for a confirmatory or more effective test. An example of the latter is a slide test which uses EBV transformed cells, the test serum and a fluorescent labelled second antibody. The transformed cells are lysed liberating EBV antigens. The slide test is reputed to be subjective, costly, requires skilled personnel and involves the potentially hazardous growth of EBV transformed cells.

Accordingly, there is a need for a diagnostic assay for EBV based on the use of bona fide EBV antigens.

A number of EBV antigens are known such as Epstein-Barr Nuclear Antigen (EBNA) and Viral Capsid Antigen (VCA) and other early EBV antigens.

The complete DNA sequence of the B95-8 strain of EBV has been determined (Baer, R., et al., (1984) Nature 310, 207-211), thereby, facilitating many molecular biology studies.

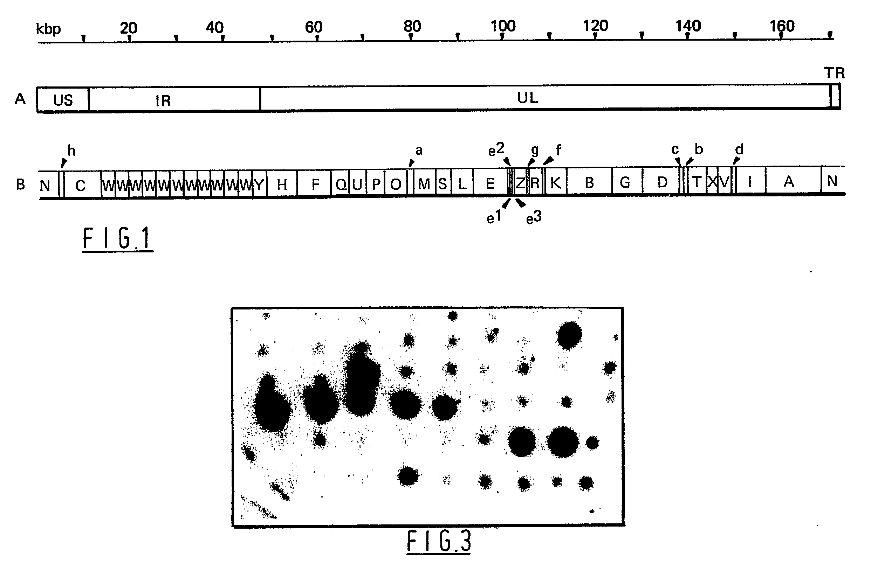

Analysis of the molecular organization of EBV has increasingly focused on that region which includes the long internal repeat sequence (see the BamHI W fragment in Fig. 1) (Cheung, A. and Kieff, E. (1982) J. Virol., 44, 286-294; Jones, M.D. and Griffin, B.E. (1983) Nucleic Acids Res., 11, 3913-3936). To date, these analyses have been of the following types:

(i) transfection studies using genomic EBV DNA fragments which have provided evidence that an intact copy of the BamHI W fragment is necessary to produce the EBV determined nuclear antigen 2 (EBNA 2) response (Rymo, L. et al., (1985) Proc. Natl. Acad. Sci. U.S.A., 82, 3435-3439; Mueller-Lantzsch, N., et al., (1985) EMBO J., 4, 1805-1811);

(ii) analysis of viral transcripts from productively and non-productively infected cells which have been shown to include sequences that have homology with the BamHI W fragment (King W. et al., (1980), J. Virol, 36, 806-818; Hummel M. and Kieff, E. (1982) J. Virol., 43, 262-272; Shin, S. et al., (1983) Virology, 124, 13-20);

(iii) the analysis and sequencing of cDNAs which potentially correspond to extensively spliced transcripts which has identified a family of cDNA clones which share common exons from BamHI W (Bodescot, M. et al., (1984) EMBO J., 3, 1913-1917; Bodescot, M. et al., (1986) Nucleic Acids Res., 14, 2611-2620; Speck, S. and Stromminger, J. (1985) Proc. Natl. Acad. Sci., U.S.A., 82, 8305-8309; Speck, S.H. et al., (1986) Proc. Natl. Acad. Sci., U.S.A., 83, 9298-9302; Sample J. et al., (1986) Proc. Natl. Acad. Sci., U.S.A. 83, 5096-5100); and

(iv) the use of synthetic peptides deduced from the predicted amino acid sequences of these repeat encoded exons which has shown that antibodies raised to some of them react with EBV expressed proteins (Dillner, J. et al., (1986) Proc. Natl. Acad. Sci., U.S.A., 83, 6641-6645).

It is an object of the present invention to provide a method enabling identification of all of the antigens from a pathogenic organism which are expressed in vivo and use of said antigens in diagnostic assays and as vaccines.

It is also an object of the present invention to provide a diagnostic assay for EBV based on bona fide EBV antigens and which has increased sensitivity or range relative to known methods for determination and detection of EBV.

Accordingly, the invention provides a method of identifying all of the antigens from a pathogenic organism which are expressed in vivo, which method comprises generating a gene bank using nucleic acid fragments which include all of the nucleic acid from a given organism and direct screening of said gene bank with serum from a subject with clinical symptoms caused by the pathogenic organism.

Preferably, the nucleic acid fragments have an average size less than 1 kb so as to avoid stop codons. In this way, all of the sequences comprising the nucleic acid of the pathogenic organism are given the opportunity to be expressed and no presumptions are made about the "appropriate" antigen or, indeed, antibody which is sought.

The invention also provides a method for obtaining nucleic acid coding for an antigen which is expressed in vivo, which method comprises converting the total nucleic acid of a pathogenic organism, which includes a sequence coding for said antigen, into nucleic acid fragments, transferring said nucleic acid fragments into an expression vector, transforming a eucaryotic or procaryotic host organism with said expression vector, screening colonies of said host organism with serum from a subject with clinical symptoms caused by the pathogenic organism.

Preferably, the nucleic acid used is DNA.

The total nucleic acid of the pathogenic organism is suitably converted into fragments using one or more restriction enzyme(s). However, one can also use various nucleases or a combination of one or more nuclease(s) and polymerase(s). The latter combination can be used to render fragments blunt-ended prior to their being ligated to nucleic acid of an expression vector. Sonication or other physical method may also be used to achieve the conversion of the total nucleic acid to nucleic acid fragments.

The pathogenic organism may be a bacterium, insect, virus or yeast or a parasite other than a bacterium, insect, virus or yeast. The pathogenic organism is preferably a virus.

The Epstein-Barr virus will be used hereinafter as a model to illustrate the invention.

The subject whose serum is screened in accordance with the invention may be a human or non-human animal.

The methods of the invention can be used to identify antigenic regions of a gene with a known protein coding region.

Any pathogenic agent will elicit an immune response. Accordingly, the methods according to the invention identify antigens which were inevitably present. The invention can be used, therefore, to isolate viral and other antigens of previously unknown molecular biology. Any nucleic acid sequence coding for a pathogenic antigen has the potential of being expressed in vivo. In most of the previous experiments in which recombinant methods were used to express an antigen in a host organism, it was necessary that the molecular biology of the nucleic acid sequence coding for the antigen was known.

The methods hereinabove specified according to the invention can be used to define the precise sequence of an antigenic region in an situation where a given gene is known to code for the antigen.

The invention also provides a method for obtaining a DNA coding for a EBV antigen as hereinafter defined, which method comprises converting EBV DNA into fragments and cloning said fragments into an expression vector, transforming a microorganism with said expression vector, screening colonies of said microorganism with sera from humans known to be infected with EBV and isolating immunopositive clones.

Preferably, the EBV DNA is digested with a restriction enzyme so as to convert it into fragments.

The method according to the invention can be used to identify a repeated sequence in a gene.

According to a further aspect of the invention there is provided a method of identifying a region which is repeated in a nucleic acid sample, which method comprises generating a gene bank using nucleic acid fragments which include all of the nucleic acid from a given organism and screening said gene bank with a probe of homologous nucleic acid and identifying strongly hybridizing colonies which are indicative of the presence of a repeated nucleic acid region.

The invention provides a DNA sequence which codes for an EBV antigen in vivo and which is selected from the following sequences:

| (A) | GC | TCC | GCC | GGG | TGG | CCC | TGG | GGT | AAG |
|---|---|---|---|---|---|---|---|---|---|
| | TCT | GGG | AGG | CAG | AGG | GTC | GGC | C | 48; |

| (B) | GC | AGC | AGG | CTC | ACC | ACC | ACA | GGC | CCC |
|---|---|---|---|---|---|---|---|---|---|
| | CCA | GAC | CCG | GGT | CTC | GGC | CAG | CCG | |
| | AGC | CGA | CCG | GCC | CCG | CGC | CTG | GCG | |
| | CCT | CCT | CGG | GGC | CAG | CCG | CCG | GGG | TTG |
| | GTT | C | | 105; | | | | | |

| (C) | C | CGG | GGT | TGG | TTC | TGC | CCC | TCT | CTC |
|---|---|---|---|---|---|---|---|---|---|
| | TGT | CCT | TCA | GAG | GAA | CCA | GGG | ACC | |
| | TCG | GGC | ACC | CCA | GAG | CCC | CTC | GGG | |
| | CCC | GCC | TCC | AGG | CGC | CCT | CCT | GGT | |
| | CTC | CGC | TCC | CCT | CTG | AGC | CCC | GTT | |
| | AAA | CCC | AAA | GAA | TGT | CTG | AGG | GGA | |
| | GCC | ACC | CTC | GGG | GCC | CAG | GCC | CCA | |
| | GAG | TC | 174; | | | | | | |

(D)  GC    CCG   AGC   CTC   TCC   CTC   GCG   GAG

AGG   GGC   29;

(E)  GG    CGC   CAA   CAG   GCC   TTT   CAG   ACC

AGG   GCG   GCG   GCT   GAA   TGC   CAT   GCC

AAA   AGC   GGG   GTG   CCG   GTC   GTG   GCC

GGC   TTC   TAC   AGG   ACC   ATC   AAC   GCC

ACG   CTC   AAG   GGA   GGA   GAG   GGC   C

117;

(F)  GT    GCC   GTG   CTA   GAT   ATT   TCA   ACT

GCC   ACA   GAC   CCC   ATT   TTG   TCC   CAC

CTG   TTA   CCA   CAT   TCT   AGG   TCC   TGC

ATC   CAG   TGG   GC    82;  and

(G)  A     GTC   CAG   ACG   CTT   TTC   CGC   CAC

GGG   GAG   CTC   TTC   CGC   TTC   ATC   TGG

GCC   CAC   TAC   GTG   AGG   C     62.

Sequences (A)-(D) are part of the BamHI W fragment of EBV and sequences (E), (F) and (G) are part of the BamHI N, BamHI F and BamHI V fragments, respectively of EBV.

Using the numbering system from Jones, M.D. and Griffin, B.E. (1983) (Nucleic Acids Res., 11, 3913-3936) for the EBV BamHI W fragment and that of Baer, R., et al., op cit for the other EBV BamHI fragments, the DNA sequences hereinabove specified have the following nucleotide numbers:

| DNA sequence | Nucleotide number |
|---|---|
| A | 2373-2421 |
| B | 2566-2671 |
| C | 2658-2832 |
| D | 2998-3027 |
| E | 2712-2831 |
| F | 55593-55674 |
| G | 146694-146755 |

The 13 bp overlap between DNA sequences (B) and (C) is underlined.

The invention also provides a DNA sequence which includes the open reading frames corresponding to sequences (A)-(G).

The invention also provides a DNA expression vector comprising a DNA sequence selected from any one of DNA sequences (A)-(G) as hereinbefore specified. The invention also provides a DNA transfer vector with expression possibilities comprising a DNA sequence selected from any one of DNA sequences (A)-(G) as hereinbefore specified. Preferably, the expression vector is a plasmid, more especially the open reading frame plasmid pORF1 (Weinstock, G.M., et al., Proc. Natl. Acad. Sci., U.S.A., 80, 4432-4436). Said expression vector may be transferred to and replicated in a microorganism.

The invention further provides a DNA sequence selected from any one of DNA sequences (A)-(G) as hereinbefore specified fused to a marker gene. Preferably said marker gene is the β-galactosidase gene of the plasmid pORF1.

Accordingly, the invention also provides a microorganism containing an expression vector comprising a DNA sequence selected from any one of DNA sequences (A)-(G) as hereinbefore specified. A particularly suitable microorganism is a bacterium such as E. coli, more especially the lac deletion strain E. coli MH3000: ara D139 Δ(ara, leu) 7697 Δ (lac) X 74 gal U gal K rps (str$^r$) ompR101 (Weinstock, G.M. et al., supra).

The invention also provides an antigenic EBV peptide/protein coded for by a DNA sequence selected from any one of DNA sequences (A)-(G) as hereinbefore specified. More particularly, the invention provides an antigenic EBV peptide/protein coded for by any one of DNA sequences (A)-(G) hereinbefore specified and selected from:

(1)

|  | | Ser | Ala | Gly | Trp | Pro | Trp | Gly | Lys |
|---|---|---|---|---|---|---|---|---|---|
| Ser | Gly | Arg | Gln | Arg | Val | Gly; | | | |

(2)

|  | | Ser | Arg | Leu | Thr | Thr | Thr | Gly | Pro |
|---|---|---|---|---|---|---|---|---|---|
| Pro | Asp | Pro | Gly | Leu | Gly | Gln | Pro | | |
| Ser | Arg | Pro | Ala | Pro | Arg | Leu | Ala | | |
| Pro | Pro | Arg | Gly | Gln | Pro | Pro | Gly | Leu | |
| Val; | | | | | | | | | |

(3)

|  | | Arg | Gly | Trp | Phe | Cys | Pro | Ser | Leu |
|---|---|---|---|---|---|---|---|---|---|
| Cys | Pro | Ser | Glu | Glu | Pro | Gly | Thr | | |
| Ser | Gly | Thr | Pro | Glu | Pro | Leu | Gly | | |
| Pro | Ala | Ser | Arg | Arg | Pro | Pro | Gly | | |
| Leu | Arg | Ser | Pro | Leu | Ser | Pro | Val | | |
| Lys | Pro | Lys | Glu | Cys | Leu | Arg | Gly | | |
| Ala | Thr | Leu | Gly | Ala | Gln | Ala | Pro | | |
| Glu; | | | | | | | | | |

(4)        Pro   Ser   Leu   Ser   Leu   Ala   Glu
    Arg  Gly;

(5)  Arg  Gln  Gln  Ala  Phe  Gln  Thr  Arg
    Ala  Ala  Ala  Glu  Cys  His  Ala  Lys
    Ser  Gly  Val  Pro  Val  Val  Ala  Gly
    Phe  Tyr  Arg  Thr  Ile  Asn  Ala  Thr
    Leu  Lys  Gly  Gly  Glu  Gly;

(6)  Ala  Val  Leu  Asp  Ile  Ser  Thr  Ala
    Thr  Asp  Pro  Ile  Leu  Ser  His  Leu
    Leu  Pro  His  Ser  Arg  Ser  Cys  Ile
    Gln  Trp;

(7)  Val  Gln  Thr  Leu  Phe  Arg  His  Gly
    Glu  Leu  Phe  Arg  Phe  Ile  Trp  Ala
    His  Tyr  Val  Arg.

The peptides may also be chemically synthesized once the nucleotide sequence of the gene coding said peptides is known.

The invention also provides a recombinant DNA expression vector comprising DNA having all or part of the nucleotide sequences (A)-(G) as hereinbefore specified or equivalent nucleotide sequences containing bases whose translated region codes for an EBV peptide/protein antigen as hereinbefore specified optionally fixed to a marker protein. Said marker protein is suitably the enzyme β-galactosidase.

The invention also provides a vaccine comprising an antigenic EBV peptide/protein as hereinbefore defined.

The invention also provides an antibody preparation specific for an EBV peptide or protein antigen as hereinbefore described.

The level of antibody against the antigens expressed in vivo can vary from one patient to another. In addition to the presence of the antigen the amount of the antigen may also be of diagnostic value. Furthermore, the class of antibody (IgM, IgA or IgG) present in the serum directed against the antigen may also be a useful diagnostic indicator.

The invention also provides a method of detecting and determining EBV, which comprises contacting serum known or suspected of containing antibody to EBV with an antigen as hereinbefore defined, allowing the immunochemical reaction of EBV antigen and antibody to EBV to take place and determining the amount of antibody to EBV present in a manner known per se. For example, antibody to EBV may be determined enzymatically, immunologically or radiometrically.

The EBV antigens according to the invention can be used as a means of obtaining and isolating specific EBV antibodies which in turn can be used to form an insoluble form of said antibodies to EBV for use in an immunoassay for the detection and determination of the corresponding EBV antigens.

The invention further provides an immunoassay method for the detection and determination of antibody to EBV which comprises:

(a) Adding a sample of a body fluid containing or suspected of containing antibody to EBV to an insolubilized form of an EBV antigen as hereinbefore defined;

(b) Allowing the immunochemical reaction to take place; and

(c) Adding a predetermined amount of a labelled antibody consisting of anti-EBV antibody covalently linked to a label and determining the activity of the reaction medium, which is a measure of the amount of EBV antibody present in the added sample.

Preferably, the labelled antibody consists of anti-EBV antibody covalently linked to an enzyme or radio label and the enzymatic or radiometric activity of the reaction medium is determined in conventional manner. The above method may be modified by adding the sample of body fluid simultaneously with a labelled anti-EBV antigen for a competitive binding immunoassay with subsequent measurement of antibody to EBV.

Alternatively for step (c) in the above method one may substitute a step comprising adding to the reaction medium a predetermined amount of anti-human immunoglobulin which binds to the antibody to EBV bound to the insolubilized EBV antigen and determining the amount of bound anti-human immunoglobulin and hence the bound EBV antibody. The anti-human immunoglobulin may be anti-IgM, anti-IgA or anti-IgG.

Preferably, the EBV antigen is coated directly or indirectly onto a surface adapted for protein adsorption. For example, said surface may be a plastics microtitration plate or strip, latex bead or "dipstick" device. Suitable microtitration plates are gamma-radiated microtitration plates such as flat-well polystyrene microtitration plates marketed by DYNATECH under the Trade Mark MICROELISA and those sold under the Trade Mark NUNC:IMMULON. Suitable microtitration strips are those sold under the Trade Mark "REMOVAWELL" by DYNATECH. The indirect coating of EBV antigen to said surface adapted for protein adsorption may be achieved by first adding anti-EBV antigen to said surface and allowing binding to occur and subsequently adding EBV antigen to said anti-EBV antigen.

However, the EBV antigens according to the invention can be used in various types of immunoassays.

Accordingly, the invention also provides an immunoassay method for the detection and determination of EBV antibody which comprises:

(a′) Adding a sample of a body fluid containing or suspected of containing EBV antibody to a surface adapted for protein adsorption as hereinabove defined;

(b′) Adding an amount of EBV antigen and allowing the immunochemical reaction to take place; and

(c′) Adding a predetermined amount of labelled anti-EBV antigen and determining the activity of the label which is a measure of the bound EBV antibody.

Alternatively, in step (b′) one can add an amount of EBV antigen fused to a reporter element such as β-galactosidase or other enzyme instead of said EBV antigen. The amount of EBV antibody can then be determined in conventional manner, for example in the case of β-galactosidase, by measuring the β-galactosidase activity of the bound EBV antigen fused to the β-galactosidase. The assay will be modified according to the reporter element used in conventional manner.

Instead of directly determining β-galactosidase activity one can also add a predetermined amount of labelled anti-β-galactosidase to the bound EBV antigen fused to β-galactosidase and determine the activity of the label as a measure of antibody to EBV. A suitably labelled antibody to β-galactosidase would comprise anti-β-galactosidase covalently linked to an enzyme label such as horse radish peroxidase. It will be appreciated that labelled antibodies to other reporter elements may also be used.

The invention also provides a method of determining different classes of antibody to EBV. For example, the invention provides an immunoassay method for the detection and determination of IgM antibody to EBV which comprises:

(a″) Adding a sample of a body fluid containing or suspected of containing IgM antibody to EBV to an insolubilized form of anti-human IgM antibody;

(b″) Allowing the immunochemical reaction to take place; and

(c″) Adding an amount of EBV antigen fused to a reporter element and allowing the further immunochemical reaction to take place.

Preferably, the reporter element is β-galactosidase.

The amount of IgM antibody to EBV is then determined either by measuring the β-galactosidase activity of the bound EBV antigen fused to β-galactosidase or other reporter element or by means of a labelled anti-β-galactosidase or other labelled antibody to a reporter element as indicated above. Alternatively, in step (c″) one can add an amount of EBV antigen instead of said EBV antigen fused to β-galactosidase. The amount of IgM antibody to EBV is then determined by the addition of labelled anti-EBV antigen, as indicated above.

The above method can be repeated for other classes of antibody to EBV such as IgA antibody to EBV antigen.

The invention also provides an immunoassay method for the detection and determination of antibody EBV which comprises:

(a‴) Adding an amount of EBV antigen fused to β-galactosidase to an insolubilized form of anti-β-galactosidase and allowing the immunochemical reaction to take place;

(b‴) Adding a sample of a body fluid containing or suspected of containing antibody to EBV and allowing the immunochemical reaction to take place; and

(c‴) Adding a predetermined amount of anti-human immunoglobulin, allowing the immunochemical reaction to take place and determining the amount of antibody to EBV by measuring the amount of bound anti-human immunoglobulin.

As in the case of the methods hereinabove specified other reporter elements may be used in place of β-galactosidase.

Steps (b‴) and (c‴) of the above method may be modified by adding the sample of body fluid simultaneously with labelled anti-EBV antigen for a competitive binding immunoasssay, thereby allowing measurement of EBV antibody.

Preferably, the sample is a serum sample.

The invention also provides a test pack for the detection and determination of EBV antibody in a body fluid, which test pack comprises:

aa A given quantity of an insolubilized EBV antigen as hereinbefore described; and

bb A corresponding quantity of the coupling product of an enzyme or radio label with an anti-EBV antibody.

In the case where the test pack contains the coupling product of an enzyme with an anti-EBV antibody, the text pack will include a substrate for the determination of the activity of said enzyme.

The radio label is preferably $^{125}$I.

However, it will be appreciated that a test pack according to the invention may comprise as appropriate any ingredient necessary for carrying out an immunoassay according to the invention as hereinabove described.

In the accompanying drawings:

Fig. 1A is a linear representation of the EBV B95-8 genome. Two clusters of tandemly repeated sequences, designated TR and IR delimit the US and UL regions;

Fig. 1B is as linear representation of the BamHI restriction map of the EBV B95-8 viral genome;

Fig. 2 depicts the expression vector pORF1 with the SmaI site into which were cloned EBV DNA fragments which include the antigen coding sequences prepared according to the invention;

Fig. 3 depicts immuno-positive clones isolated following the screening of the EBV B95-8 genomic bank prepared in accordance with the invention with a pool of anti-EBV human sera;

Fig. 4 depicts plasmid DNA prepared from a selection of the immuno-positive clones depicted in Fig. 3 following digestion with ClaI;

Fig. 5 is an autoradiograph of a Southern blot of the plasmid DNA of Fig. 4 probed with a $^{32}$p-labelled 803 bp PvuII subfragment of BamHI of EBV B95-8;

Fig. 6 is a schematic representation of a single copy of an EBV B95-8 BamHI W fragment showing the relative positions and reading frames of sequences known to be transcribed and/or expressed - the thick black horizontal bars identified as clones A to D (CL A, CL B, CL C and CL D), represent the expressed DNA sequences (A)-(D) which code for EBV antigens in accordance with the invention; and

Fig. 7 is a linear representation of the EBV B95-8 genome indicating the relative positions of DNA sequences (A)-(G) which code for EBV antigens in accordance with the invention.

The invention will be further illustrated by the following Examples.


Example 1


Isolation of clones that express EBV antigens

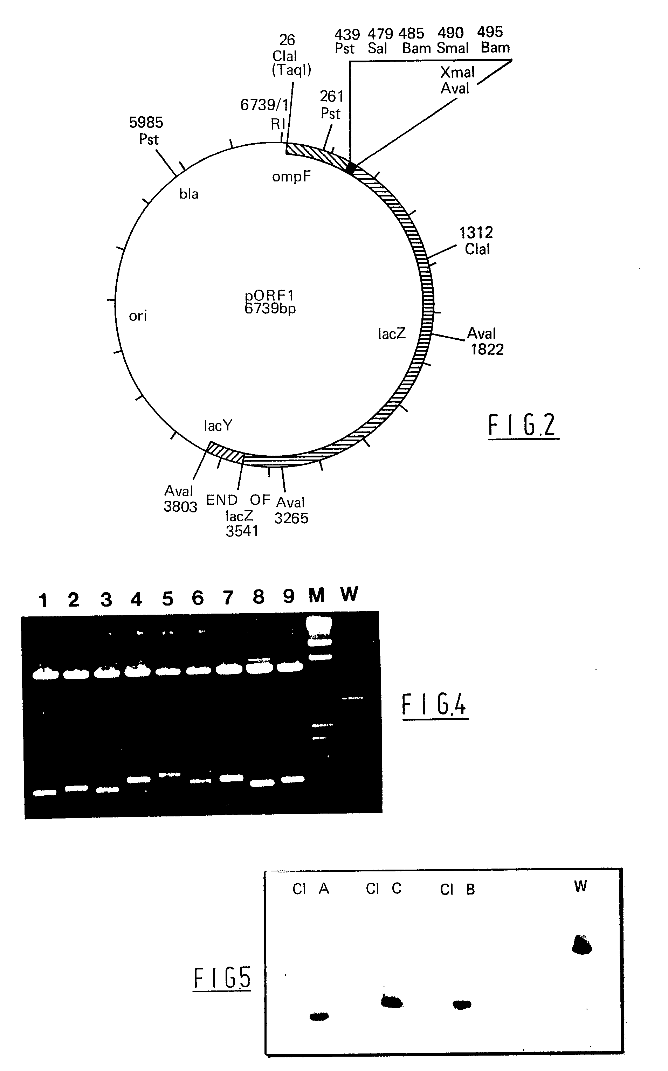

Commercial EBV B95-8 DNA which was purchased from two sources: (i) The University Research Institute for Biomedicine in Florida, U.S.A. and (ii) Microbiological Associates Inc., Bethesda, Maryland, U.S.A. and which was received partially degraded to an average fragment size of less than 1 kb was made blunt-ended by using a combination of S1 nuclease and T4 DNA polmerase and ligated to the unique SmaI site of the open reading frame expression vector pORF1 (see Fig. 2) (Weinstock et al., (1983) supra). The relevant characteristics of pORF1 are that (i) it contains a β-galactosidase gene that is inactive; (ii) it contains a polylinker cloning site at the N-terminal end of the β-galactosidase gene and (iii) ORF DNA inserted into this site is potentially expressed under the control of an E. coli promotor and initiator sequence ompF. An appropriate change in the translation phase by the insert will result in the expression of β-galactosidase possibly as a fused protein.

The expression vector containing the EBV DNA was transformed into the lac deletion strain E. coli MH3000 referred to above. The expression vector pORF1 and the E. coli MH3000 were gifts from G. M. Weinstock. Sequence (G) has also functioned in E. coli strain J.M. 105. Samples of E. Coli with plasmids containing the EBV DNA and designated pEBV-A, pEBV-B, pEBV-C, pEBV-D, pEBV-E, pEBV-F and pEBV-G were deposited at The National Collections of Industrial and Marine Bacteria Limited (NCIMB Limited) on October 24, 1988 and accorded the numbers NCIB 40075, NCIB 40076, NCIB 40077, NCIB 40078, NCIB 40079, NCIB 40080 and NCIB 40081, respectively.

Plasmid DNA was prepared by standard CsCl/ethidium bromide centrifugation method (Maniatis, T. et al., (1982) Molecular Cloning, a Laboratory Manual - Cold Spring Harbour, Cold Spring Harbour, New York). Alternatively, plasmid DNA can be prepared from minilysates using the alkaline lysis procedure (Birnboim, H.C. and Doly, J. (1979) Nucleic Acids Res. 7, 1513-1523).

The transformation procedure used was that according to Dagert, M. and Erlich, S.D. (1979) Gene 6, 23. Transformants were plated on media containing ampicillin and 5-bromo-4-chloro-3-indolyl-β-D-galactopyranoside.

The gene bank, which comprised 60,000 recombinants was screened for the expression of EBV antigens using pooled sera from IM patients. Serum was taken from patients diagnosed as having infectious mononucleosis at the Regional Hospital, Galway, Ireland. The gene bank is described also in Nucleic Acids Res. 16, 7 (Walls, D. et al., 1988).

Diagnosis was based on the determination of antibody levels to viral capsid (VCA) and early (EA) antigens of EBV by indirect immunofluorescence (Henle, W. and Henle, G. (1966) J. Bacteriol., 91, 1248-1256) on slides containing P3HR-1 and Raji cells, respectively and induced by 12-0-tetradecanoylphorbol-13-acetate. Antibody titers to the EBV nuclear antigens (EBNA) were determined on Raji cells by ACIF (Reedman, B.M. and Klein, G. (1973) Int. J. Cancer, 11, 499-520). Titer estimations were 1:512 for VCA and 1:256 for EBNA. Titers for antibodies to EA were not quantified but rather were scored as + or -, with most sera scoring +.

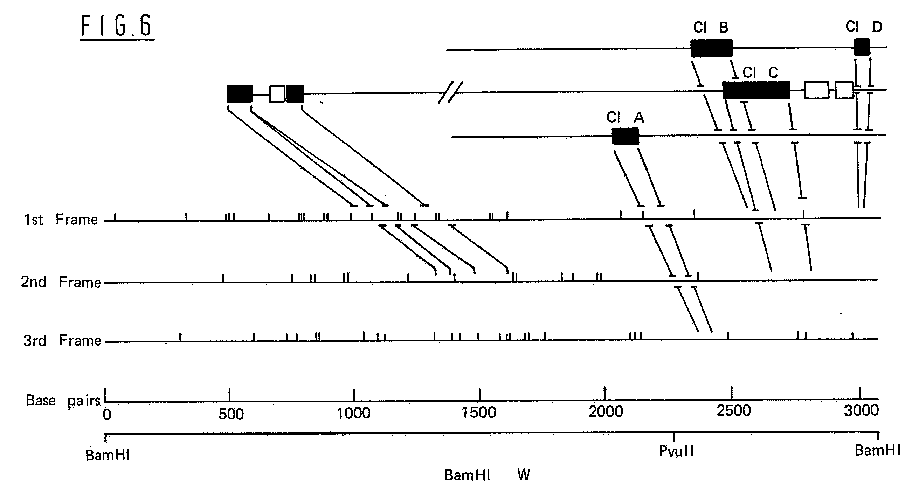

Pretreatment of the serum proved necessary to reduce an initially heavy background possibly due to antibodies cross-reacting with E. coli proteins. Such cross-reacting proteins were removed in the pretreatment step by passing the pooled sera through an affinity chromatography column having E. coli proteins bound thereto. Positive clones isolated in the screening step are shown in Fig. 3.

Colonies to be screened were lysed in situ on nitrocellulose filters as described by Young, R.A. and Davies, R.W. (1983) Proc. Natl. Acad. Sci., U.S.A., 80, 1194-1196 with minor modifications by Walls, D. et al., (1988) (Nucleic Acids Res. 16, 7) in the following manner:

Procedure:

Various buffers were prepared as follows:

| | |
|---|---|
| Buffer A1: | 0.17 M NaCl |
| | 0.01 M Tris-HCl (pH 8.0) |
| | 0.1 mM PMSF (phenylmethanesulphonyl fluoride) |
| | 1.0 mM KI |
| Buffer A2: | Buffer A1 + 0.01% SDS (sodium dodecyl sulphate) |
| Buffer A3: | Buffer A1 + 3% BSA bovine serum albumin) (or 5% milk protein (Cadbury's Marvel: Marvel is a Trade Mark)) + 0.1% sodium azide. (Prepared on day of experiment). |
| Buffer B1: | Buffer A1 + 0.1% Triton (Triton is a Trade Mark) X100, 1mM EDTA. |
| Buffer B2: | Buffer B1 + 0.1% SDS. |
| Buffer B3: | Buffer B1 + 3% BSA (or 5% Marvel) + 0.1% sodium azide. (Prepared on day of experiment). |

Treatment of Serum

An overnight culture of host cells (1.5 ml) (see step (1) below) was centrifuged at 6,000 rpm for 5 min. The supernatant was removed and the pellet resuspended in PBS (phosphate buffered saline; 1 tablet/100 ml H$_2$O) (100 µl). PBS (90 µl) was added to a sample of serum (10 µl) and the serum so prepared was added to resuspended host cells (100 µl) and left at room temperature for 30-45 min. The sample was then centrifuged at 8,000 rpm for 5 min. The supernatant was ready for adding to the filters containing the B3 buffer (see step (9) below).

Immunological screening of the expression bank with human anti-EBV sera was then performed over a period for 4 days in the following way:

Day 1

(1) Colonies were grown on appropriate selection media overnight at 37°C. Replica copies of the colonies were made on another plate.

Day 2

(2) A nitrocellulose filter was placed on the plate containing the colonies and left for 5 min. The nitrocellulose filter colony left was placed on an 82 mm Whatman 541 filter soaked in buffer A1 and lysozyme (2 mg/ml) in the base of a petri dish. A Whatman 541 filter soaked in chloroform was placed in the lid of the petri dish and the dish was closed and the assembly incubated for 30 min. at room temperature resulting in lysis of the clones.

(3) The filter was then removed and soaked, colony side up, in a new dish containing buffer A2 (7 ml) for 1 h. This step and all subsequent steps were carried out at room temperature.

(4) The filter was removed and rinsed, colony side up, in buffer A1 (7 ml) for 10 min.

(5) The filter was removed and incubated as before in buffer A1 (7 ml) containing DNAse (2 µg/ml).

(6) The filter was removed and rinsed in buffer A1 (7 ml) for 10 min. The filter was then transferred to a glass petri dish. Glass petri dishes were used in all subsequent steps.

(7) The filter was incubated in buffer A3 (7 ml/filter) for 1 h. While this step was in progress the treatment of serum referred to above was carried out.

(8) The sera (supernatant from treatment procedure) was diluted in buffer B3 (7 ml) and the filter incubated therein. The filter was then incubated overnight with gentle agitation.

## Day 3

(9) The buffer containing the serum was removed and the filter was washed in buffer B2 (7 ml) for 10 min.

(10) The filters were washed in buffer B1 (7 ml for 10 min.).

(11) Step (10) was repeated.

(12) The filters were washed in buffer B3 (7 ml) for 15 min.

(13) The filters were incubated with $^{125}I$ protein A (Sigma) at approximately $2 \times 10^6$ cpm/filter (diluted in buffer B3) overnight.

## Day 4

(14) Step (9) was repeated.

(15) The filters were washed in buffer B1 (7 ml) for 15 min.

(16) Step (15) was repeated for three further 15 min. periods.

(17) The filters were dried and autoradiographed with intensifying screen for 12-48 h. at -70°C resulting in detection of bound antibody.

## Example 2

### Identification of clones that express EBV antigens encoded by BamHI W.

The EBV DNA in colonies selected and purified as having positive antigen expression, namely antigens which correspond to antibodies present in the pooled serum, was sequenced and the location of the open reading frames identified on the EBV gene map.

To prepare a probe for the BamHI W region, intact commercial EBV B95-8 DNA was digested with BamHI and cloned into pORF1. This bank was screened with degraded total EBV DNA as probe. Colonies to be screened were transferred according to manufacturers' instructions onto 82 mm nitrocellulose filters (Schleicher and Schuell) and the hybridization protocol of the manufacturer was followed. Southern blot analysis of the digested plasmids was performed on Hybond N (Trade Mark - Amersham) using 0.4 M NaOH as transfer buffer. DNA probes were generated by the oligolabelling procedure (Feinberg, A.P. and Vogelstein, B. (1983) Analytical Biochem., 132, 6-13). Strongly hybridizing colonies were picked for analysis and most were found to contain pORF1 recombinants with a copy of EBV BamHI W as shown by size and confirmed by subsequent results. For the purposes of this study, an 803 bp PvuII-BamHI subfragment of W (Fig. 6) was isolated, radiolabelled and used to probe ClaI digestions of plasmid DNA minipreparations from the immuno-positive clones. The results are shown in Figs. 4 and 5.

Positive hybridization can be seen from the plasmid DNA of three immuno-positive clones. Referring specifically to Fig. 4, cloned viral DNA fragments are attached to a 1.3 kb vector sequence and their presence can be implied as an increase in the size of this fragment. Lane 1 contains pORF1 and lanes 2-9 contain pORF1 recombinants from immuno-positive clones. Lane M contains λ size markers (HindIII digest) and Lane W contains purified EBV B95-8 BamHI W. Fig. 5 is an autoradiograph of a Southern blot of the gel shown in Fig. 4 probed with a $^{32}$p-labelled 803 bp PvuII subfragment of BamHI W. Positive hybridization can be seen from three clones, CL A, CL B and CL C and from BamHI W itself.

## Example 3

### DNA Sequence of BamHI W Antigen expressing Fragments

Inserts in pORF1 were subcloned as BamHI fragments into the sequencing vector M13 mp10 (Amersham) and sequenced by the di-deoxy method. DNA sequencing data were analysed using the Microgenie (Trade Mark) software package and the Genbank (Trade Mark) data base. Comparison of these sequences with the known EBV B95-8 sequence (Baer et al., (1984) supra) has allowed the determination of their exact location in BamHI W. All of the sequences are part of open reading frames which are in the same direction of translation as they were found in the expression vector. When aligned together on one copy of the repeat, however, these sequences can be seen to be parts of three different open reading frames with two of the fragments containing a 13 bp overlap (Fig. 6). The locations of DNA sequences previously identified and characterised cDNAs (Bodescot, M. et al., (1984) supra; Bodescot, M. et al., (1986) supra; Speck, S. et al., (1986) supra; Sample, J. et al., (1986) supra) from this region and sequences proven to be antigen coding are also indicated in Fig. 6 to highlight the fact that the DNA sequences according to the invention are not known to be part of these genes.

Referring to Fig. 6, the first, second and third frames indicated refer to the three possible reading frames as shown from left to right on the standard physical map depicted in Fig. 1. Thin vertical lines show the location of termination codons. As indicated above, the thick black horizontal bars represent known expressed sequences: these include DNA sequences (A)-(D) according to the invention and other sequences shown to be antigenic (Dillner, J., et al., (1986) Proc. Natl. Acad. Sci., U.S.A., 83, 6641-6645). Sequences represented by white horizontal bars have been deduced not to be antigenic-coding (Dillner, J. et al., (1985) EMBO J., 4, 1813-1818 and Dillner, J. et al., (1986) supra). W1 and W2 indicate exons shown to be present in a large family of cDNA's (Bodescot, M., et al., (1984) supra; Speck, S. and Strominger, J. (1985) supra; Sample J., et al., (1986) supra). The baseline of the drawing shows the position of the PvuII site used to generate the 803 bp subfragment (base numbers 2269-3072) of BamHI W used as a probe for clones CL A-CL D in Example 2. It is therefore to be noted that this probe does not contain sequences from exons W1 and W2 described previously.

The procedure used in the above Examples is also described by the inventors in the EMBO Journal 7, 4, pp 1191-1196 (1988).

In connection with the present invention, the Applicants have sought to identify EBV ORF's from the repeated BamHI W region and other regions of EBV which, in fact, code for antigenic peptide/protein fragments in vivo. This approach distinguishes itself from other analyses of the region by using random fragments from total EBV DNA which were subcloned into an expression vector and by screening this gene bank with serum from EBV immuno-positive patients. While most studies on EBV gene expression have been based on viral propagation and maintenance in cell culture, the experimental design used in connection with the present invention should reflect the situation in vivo. Using a combination of immunodetection and nucleic acid hybridization, the Applicants have isolated seven DNA fragments which by the design of the above experiments must be expressed and be antigenic in vivo. It is anticipated that further DNA fragments can be similarly isolated. The 7 EBV antigens identified herein are not part of proteins previously described as EBV antigens and may be used to diagnose the presence of antibodies to EBV in a sample. A detailed clinical study is required to establish any correlation between the presence, level and class of these antigens and any pathologies attributed to EBV.

Referring to Fig. 6 of the drawings, the large internal repeat in EBV B95-8 is repeated about eleven times in the virus and as a consequence, it is impossible to assign any of the sequences (A)-(D) presented hereinabove to any one of them. However, fragments from all of CL A to CL D are part of different ORF's if one accepts that the termination codon between CL B and CL D is effective. The three different potential reading frames in that region are also used. Given the current understanding of the potential for extensive splicing in that region of EBV (Bodescot, M., et al., (1984, 1986) supra; Speck, S. and Strominger, J., (1985) supra; Speck et al., (1986) supra; Sample, J. et al., (1986) supra) it is possible that some of these clones code for different parts of the same protein.

Two of the clones CL B and CL C contain overlapping sequences. As they are also in different ORFs, they probably represent parts of different proteins. This would clearly indicate that the same sequence could be part of different exons arising probably from different splicing patterns. Such a sharing of exons by EBV proteins has previously been reported for some of the EBNA proteins (Bodescot, M. et al., (1984) supra). The present invention suggests that this may be a common occurrence in this system. When this fact is considered in conjunction with the diverse possibilities implicit in a repeated region, which may not necessarily be spliced in an identical manner in every repeat, it becomes evident that a potential ORF may in fact be expressed, may occasionally be expressed or may never be expressed.

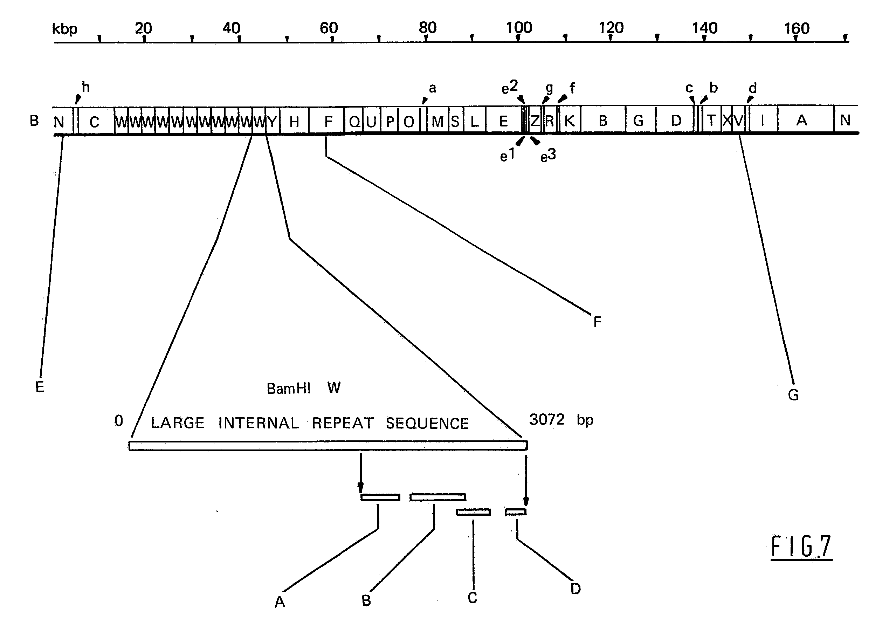

Because of the design of the above experiments, it is possible that the protein of which CL B is part is expressed in one group of cells or tissues and that the protein which includes CL C is expressed in another. If both are antigenic, the sera which were used in connection with the present invention would include antibodies to both. A preliminary experiment carried out in connection with the present invention, in which individual rather than pooled sera were used in the immuno-detection of EBV antigens in selected colonies, showned no detectable difference in the signal obtained for clones CL A-CL F, although some fluctuation was observed with CL G (Fig. 7), the levels of binding found in EBV antigen expressing clones from other regions of the virus did fluctuate (results not shown).

The above Examples clearly shown that DNA sequences (A)-(G) can be expressed by EBV during infectious mononucleosis. In recent Northern analysis experiments on RNA prepared from the EBV containing cell lines B95-8, Raji and Namalwa have failed to detect any transcripts homologous to the PvuII-BamHI subfragment of the large internal repeat sequence or to the sequences found in CL F. In a follow-up experiment, it was not possible to isolate clones containing sequences from this region from a cDNA bank prepared from the polyadenylated cytoplasmic RNAs of the EBV B95-8 cell line (a gift from M. Perricaudet, see Bodescot et al., (1984, 1986) supra). All of these results taken together suggest that the viral sequences CL A-CL D and CL F described herein are expressed in one group of cells or tissues and that cell culture may not have the same set of factors, which may modulate the expression of EBV. Alternatively, it may have been the case that a strain of EBV, which expresses the sequences presented herein, is widely present in the population used as a source of serum. In similar experiments with the mRNA population of cell lines containing EBV B95-8 DNA both CL E and CL G did positively identify corresponding cDNA sequences.

As indicated above, a large EBV B95-8 genomic DNA bank has been prepared in accordance with the invention in an E. coli expression vector and screened with a pool of sera from human IM patients. Immuno-positive clones thus obtained were subsequently screened with a probe for the W region. Four of the

EP 0 316 170 A2

immuno-positive clones which also contained sequences from the viral large internal repeat and three immuno-positive clones from other regions of EBV were selected. DNA sequence analysis of said four clones which code for the immuno-detected product of a BamHI W open reading frame (ORF) has located them on the repeat sequence and has shown that they come from three potential open reading frames and that two of them consist of overlapping reading frames as indicated above. This must imply extensive intron/exon splicing or that the repeat itself encodes several different proteins. The seven expressed epitopes were shown to be present simultaneously in independent cases of infectious mononucleosis. Many of these (CL A-CL D and CL F) epitopes have not been previously described and based on the experiments design, used in connection with the present invention, it must reflect the situation in vivo.

None of the DNA sequences (A)-(G) prepared in accordance with the invention codes for parts of any of the previously described EBNA proteins nor are CL A-CL D present in any cDNAs found to date which correspond to highly spliced transcripts that span the major internal repeat region. The failure to detect them in a well characterised cDNA bank prepared from B95-8 RNA suggest that they may be expressed in vivo in man but not under the conditions used in cell culture.

## Claims

1. A method of identifying all of the antigens from a pathogenic organism which are expressed in vivo, which method comprises generating a gene bank using nucleic acid fragments which include all of the nucleic acid from a given organism and direct screening of said gene bank with serum from a subject with clinical symptoms caused by the pathogenic organism.

2. A method for obtaining nucleic acid coding for an antigen which is expressed in vivo, which method comprises converting the total nucleic acid of an pathogenic organism, which includes a sequence coding for said antigen, into nucleic acid fragments, transferring said nucleic acid fragments into an expression vector, transforming a eucaryotic or procaryotic host organism with said expression vector, screening colonies of said eucaryotic or procaryotic organism with serum from a subject with clinical symptoms caused by the pathogenic organism.

3. A method of identifying a region which is repeated in a nucleic acid sample, which method comprises generating a gene bank using nucleic acid fragments which include all of the nucleic acid from a given organism and screening said gene bank with a probe of homologous nucleic acid and identifying strongly hybridizing colonies which are indicative of the presence of a repeated nucleic acid region.

4. A method for obtaining a DNA coding for an EBV antigen expressed in vivo, which method comprises converting EBV DNA into fragments and cloning said fragments into an expression vector, transforming a microorganism with said expression vector, screening colonies of said microorganism with sera from humans known to be infected with EBV and isolating immunopositive clones.

5. A DNA sequence which codes for an EBV antigen in vivo and which is selected from the following sequences:

13

(A)  GC   TCC   GCC   GGG   TGG   CCC   TGG   GGT   AAG
     TCT   GGG   AGG   CAG   AGG   GTC   GGC   C   48;


(B)  GC   AGC   AGG   CTC   ACC   ACC   ACA   GGC   CCC
     CCA   GAC   CCG   GGT   CTC   GGC   CAG   CCG
     AGC   CGA   CCG   GCC   CCG   CGC   CTG   GCG
     CCT   CCT   CGG   GGC   CAG   CCG   CCG   GGG   TTG
     GTT   C   105;


(C)  C   CGG   GGT   TGG   TTC   TGC   CCC   TCT   CTC
     TGT   CCT   TCA   GAG   GAA   CCA   GGG   ACC
     TCG   GGC   ACC   CCA   GAG   CCC   CTC   GGG
     CCC   GCC   TCC   AGG   CGC   CCT   CCT   GGT
     CTC   CGC   TCC   CCT   CTG   AGC   CCC   GTT
     AAA   CCC   AAA   GAA   TGT   CTG   AGG   GGA
     GCC   ACC   CTC   GGG   GCC   CAG   GCC   CCA
     GAG   TC 174;


(D)  GC   CCG   AGC   CTC   TCC   CTC   GCG   GAG
     AGG   GGC   29;


(E)  GG   CGC   CAA   CAG   GCC   TTT   CAG   ACC
     AGG   GCG   GCG   GCT   GAA   TGC   CAT   GCC
     AAA   AGC   GGG   GTG   CCG   GTC   GTG   GCC
     GGC   TTC   TAC   AGG   ACC   ATC   AAC   GCC
     ACG   CTC   AAG   GGA   GGA   GAG   GGC   C
     117;

14

| (F) | GT | GCC | GTG | CTA | GAT | ATT | TCA | ACT |
| | GCC | ACA | GAC | CCC | ATT | TTG | TCC | CAC |
| | CTG | TTA | CCA | CAT | TCT | AGG | TCC | TGC |
| | ATC | CAG | TGG | GC | 82; and | | | |

| (G) | A | GTC | CAG | ACG | CTT | TTC | CGC | CAC |
| | GGG | GAG | CTC | TTC | CGC | TTC | ATC | TGG |
| | GCC | CAC | TAC | GTG | AGG | C | 62. | |

6. A DNA expression vector or a DNA transfer vector with expression possibilities comprising a DNA sequence according to Claim 5.

7. A microorganism containing a vector as claimed in Claim 6.

8. An antigenic EBV peptide or protein coded for by a DNA sequence as claimed in Claim 5 and comprising an amino acid sequence selected from:

| (1) | | Ser | Ala | Gly | Trp | Pro | Trp | Gly | Lys |
| | Ser | Gly | Arg | Gln | Arg | Val | Gly; | | |

| (2) | | Ser | Arg | Leu | Thr | Thr | Thr | Gly | Pro |
| | Pro | Asp | Pro | Gly | Leu | Gly | Gln | Pro | |
| | Ser | Arg | Pro | Ala | Pro | Arg | Leu | Ala | |
| | Pro | Pro | Arg | Gly | Gln | Pro | Pro | Gly | Leu |
| | Val; | | | | | | | | |

| (3) | | Arg | Gly | Trp | Phe | Cys | Pro | Ser | Leu |
| | Cys | Pro | Ser | Glu | Glu | Pro | Gly | Thr | |
| | Ser | Gly | Thr | Pro | Glu | Pro | Leu | Gly | |
| | Pro | Ala | Ser | Arg | Arg | Pro | Pro | Gly | |
| | Leu | Arg | Ser | Pro | Leu | Ser | Pro | Val | |
| | Lys | Pro | Lys | Glu | Cys | Leu | Arg | Gly | |

|     | Ala | Thr | Leu | Gly | Ala | Gln | Ala | Pro |
|-----|-----|-----|-----|-----|-----|-----|-----|-----|
|     | Glu; |    |     |     |     |     |     |     |

| (4) |     | Pro | Ser | Leu | Ser | Leu | Ala | Glu |
|-----|-----|-----|-----|-----|-----|-----|-----|-----|
|     | Arg | Gly; |    |     |     |     |     |     |

| (5) | Arg | Gln | Gln | Ala | Phe | Gln | Thr | Arg |
|-----|-----|-----|-----|-----|-----|-----|-----|-----|
|     | Ala | Ala | Ala | Glu | Cys | His | Ala | Lys |
|     | Ser | Gly | Val | Pro | Val | Val | Ala | Gly |
|     | Phe | Tyr | Arg | Thr | Ile | Asn | Ala | Thr |
|     | Leu | Lys | Gly | Gly | Glu | Gly; |    |     |

| (6) | Ala | Val | Leu | Asp | Ile | Ser | Thr | Ala |
|-----|-----|-----|-----|-----|-----|-----|-----|-----|
|     | Thr | Asp | Pro | Ile | Leu | Ser | His | Leu |
|     | Leu | Pro | His | Ser | Arg | Ser | Cys | Ile |
|     | Gln | Trp; |    |     |     |     |     |     |

| (7) | Val | Gln | Thr | Leu | Phe | Arg | His | Gly |
|-----|-----|-----|-----|-----|-----|-----|-----|-----|
|     | Glu | Leu | Phe | Arg | Phe | Ile | Trp | Ala |
|     | His | Tyr | Val | Arg. |   |     |     |     |

9. A vaccine comprising an antigenic EBV peptide or protein as claimed in Claim 8.

10. An antibody preparation specific for an EBV peptide or protein antigen as claimed in Claim 8.

11. A method of detecting and determining EBV, which comprises contacting serum known or suspected of containing antibody to EBV with an EBV peptide or protein antigen claimed in Claim 8, allowing the immunochemical reaction of EBV antigen and antibody to EBV to take place and determining the amount of antibody to EBV present in a manner known per se.

12. An immunoassay method for the detection and determination of antibody to EBV which comprises:

(a) Adding a sample of a body fluid containing or suspected of containing antibody to EBV to an insolubilized form of an EBV antigen as claimed in Claim 8.

(b) Allowing the immunochemical reaction to take place; and

(c) Adding a predetermined amount of a labelled antibody consisting of anti-EBV antibody covalently linked to a label and determining the activity of the reaction medium, which is a measure of the amount of EBV antibody present in the added sample.

13. An immunoassay method for the detection and determination of antibody to EBV which comprises:

(a*) Adding a sample of a body fluid containing or suspected of containing antibody to EBV to an insolubilized form of an EBV antigen as claimed in Claim 8.

(b*) Allowing the immunochemical reaction to take place; and

(c*) Adding to the reaction medium a predetermined amount of anti-human immunoglobulin which binds to the antibody to EBV bound to the insolubilized EBV antigen and determining the amount of bound anti-immunoglobulin and, thereby, the bound EBV antibody.

14. An immunoassay method for the detection and determination of EBV antibody which comprises:

(a') Adding a sample of a body fluid containing or suspected of containing EBV antibody to a surface adapted for protein adsorption;

(b') Adding an amount of EBV antigen and allowing the immunochemical reaction to take place; and

(c') Adding a predetermined amount of labelled anti-EBV antigen and determining the activity of the label which is a measure of the bound EBV antibody.

15. A method according to Claim 14, wherein an amount of EBV antigen fused to a reporter element is added instead of said EBV antigen and the amount of EBV antibody is determined in a manner known per se.

16. An immunoassay method for the detection and determination of IgM antibody to EBV which comprises:

(a″) Adding a sample of a body fluid containing or suspected of containing IgM antibody to EBV to an insolubilized form of anti-human IgM antibody;

(b″) Allowing the immunochemical reaction to take place; and

(c″) Adding an amount of EBV antigen fused to a reporter element and allowing the further immunochemical reaction to take place.

17. A test pack for the detection and determination of EBV antibody in a body fluid, which test pack comprises:

aa A given quantity of an insolubilized EBV antigen as claimed in Claim 8; and

bb A corresponding quantity of the coupling product of an enzyme or radio label with an anti-EBV antibody.

EP 0 316 170 A2

kbp    20    40    60    80    100    120    140    160

TR

A   | US | IR | UL |

h      a    e2   g   f     c   b    d

B   N | C | WWWWWWWWWWWWWWY | H | F | Q | U | P | O | M | S | L | E | Z | R | K | B | G | D | T | X | V | I | A | N

e1    e3

**F I G.1**

**F I G.3**

F I G.2

F I G.4

F I G.5

F I G. 6

CI B    CI D
CI C
CI A

1st Frame

2nd Frame

3rd Frame

Base pairs
0      500     1000    1500    2000    2500    3000

BamHI                                    PvuII          BamHI

BamHI    W

EP 0 316 170 A2

EP 0 316 170 A2

kbp    20    40    60    80    100    120    140    160

B  N │ C │WWWWWWWWWWWW│Y│ H │ F │Q│U│P│O│M│S│L│ E │Z│R│K│ B │ G │ D │T│XV│ I │ A │ N

h    a    e2  g  f    c  b    d

e1    e3

E

F

G

BamHI    W

0 / LARGE INTERNAL REPEAT SEQUENCE    3072 bp

A    B    C    D

FIG.7